# EUROPÄISCHE PATENTSCHRIFT

(11) **EP 0 702 944 B1**
(45) Veröffentlichungstag und Bekanntmachung des Hinweises auf die Patenterteilung: **05.01.2000**
(21) Anmeldenummer: 94810559.8
(22) Anmeldetag: 26.09.1994
(51) Int. Cl.: A61F 6/14

(54) **Okklusivpessar**
Occlusive pessary
Pessaire occlusif

(43) Veröffentlichungstag der Anmeldung: 27.03.1996
(73) Patentinhaber: Cimber, Hugo, Dr. med., CH-3072 Ostermundigen (CH)
(72) Erfinder: Cimber, Hugo, Dr. med., CH-3072 Ostermundigen (CH)
(74) Vertreter: Fischer, Franz Josef

(56) Entgegenhaltungen:
- EP-A- 0 208 653
- WO-A-91/00714
- US-A- 3 880 156

## Beschreibung

Die vorliegende Erfindung bezieht sich auf einen Okklusivpessar, der für den Einsatz unmittelbar nach einer Schwangerschaft geeignet ist, gemäss dem Oberbegriff des Patentanspruches 1.

Es sind Okklusivpessare bekannt, bei welchen zwei voneinander weg aufspreizbare Äste aus einem Röhrchen ausgestossen werden, wobei die Äste an ihren Enden Abschlussorgane tragen. So ist beispielsweise aus der FR-A-2 085 578 ein Okklusivpessar bekannt, der mittels eines Röhrchens in die Gebärmutter eingeführt wird, wobei ein Tragbalken mit zwei an dessen in der Einführrichtung vorderen Ende angelenkten Ästen vorgesehen ist, derart, dass sich die Äste nach ihrem Ausstossen aus dem Röhrchen seitlich voneinander wegspreizen. An ihren freien Enden tragen die Äste je ein kugeliges Abschlussorgan, welche Abschlussorgane dazu bestimmt und geeignet sind, die Mündungen der Eileiter in die Gebärmutter zu verschliessen. Die Äste sind von ungefähr gleicher Länge, so dass die beiden Abschlussorgane beim Einziehen des Pessars in das Röhrchen nebeneinander zu liegen kommen und das Röhrchen somit einen vergleichsweise grossen Durchmesser aufweisen muss. Gemäss der EP-A-0 208 653 geht das Bestreben bei Okklusivpessaren dahin, den Durchmesser des Röhrchens möglichst klein zu halten, da bekanntlich die Einführung solcher Röhrchen umso leichter und angenehmer wird, je kleiner der Röhrchendurchmesser ist. Dieser Durchmesser wiederum hängt im wesentlichen davon ab, in welcher gegenseitigen Stellung die am Ende der genannten Äste befestigten Abschlussorgane in das Röhrchen eingezogen werden können. Im Gegensatz zum erstgenannten französischen Dokument ist in der zweitgenannten Schrift vorgesehen, dass einer der Äste mittels mindestens einer Sollknickstelle derart versehen ist, dass die kugeligen Abschlussorgane bei in das Röhrchen eingezogenen Ästen in der Einzugsrichtung gesehen hintereinander und nicht nebeneinander zu liegen kommen. Bisher musste mit dem Einführen eines Okklusivpessars bei Frauen, die unmittelbar eine Geburt hinter sich hatten ca. 6 Wochen zugewartet werden, damit sich die Gebärmutter wiederum in ihre ursprüngliche Grösse zurückbilden konnte, weil sonst der Pessar mit grosser Wahrscheinlichkeit wieder ausgestossen wurde. Es besteht somit ein Bedürfnis nach einem Pessar dessen Einführung unmittelbar nach der Geburt vorgenommen werden kann. Dies war mit den bisher bekannten Pessaren nicht möglich, da der Abstand der Abschlussorgane der vorbekannten Pessare dem Abstand der Eileitermündungen im zurückgebildeten Zustand der Gebärmutter entspricht. Unmittelbar nach der Geburt ist die Gebärmutter ca. 12 cm breit und 17 cm lang; die Öffnung des Muttermunds hat dabei einen Durchmesser von 3 bis 4 cm. Im zurückgebildeten Zustand, ca. 6 Wochen nach der Geburt ist die Gebärmutter ca. 4 cm breit und 7 cm lang und die Öffnung des Muttermundes beträgt 3-4 mm. Unmittelbar nach der Geburt wären somit die Voraussetzungen für eine Einführung eines Pessar wegen dem offenen Muttermund sehr günstig, da diese im Rahmen einer abschliessenden Untersuchung der Frau vor der Entlassung aus der Klinik durchgeführt werden kann. Der Pessar muss hierzu derart ausgebildet sein, dass er seine Aufgabe auch während den 6 Wochen der Zurückbildung der Gebärmutter erfüllen kann, d.h. er muss nach seiner Einführung in der korrekten Position bleiben und gleichzeitig müssen die Abschlussorgane ihre Verschlussfunktion ausüben. Bisher sind keine derartigen Pessare bekannt.

Das Ziel der vorliegenden Erfindung ist es somit, einen Okklusivpessar zu schaffen, der so ausgebildet ist, dass er im Vergleich mit dem bekannten Stand der Technik unmittelbar nach einer Schwangerschaft eingeführt werden kann, wobei sich ein Positionierungs- und Haltemittel gleichzeitig mit der Zurückbildung der Gebärmutter den sich verkleinernden Dimensionen anpasst.

Diese Aufgabe wird erfindungsgemäss mit einem Okklusivpessar gelöst, der die Merkmale des kennzeichnenden Teiles des Patentanspruches 1 aufweist.

Bevorzugte Ausführungsformen sind durch die Merkmale der Patentansprüche 2 bis 5 gekennzeichnet.

Der Halte- und Positionierungsbalken aus resorbierbarem oder biologisch abbaubarem Material, der während der Rückbildung der Gebärmutter durch seine Positioniereungs-Enden gegebenenfalls auch vorläufige Abschlussorgane für die Eileitermündungen zur Verfügung stellt, besitzt eine Länge von ca. 12 cm (enspricht der Breite der Gebärmutter nach der Geburt) und ist an den definitiven Abschlussorganen befestigt, derart, dass diese nach dem Abbau des Halte- und Positionierungsbalkens ihre Funktion übernehmen können. Seine beiden Positionierungs-Enden sind vorteilhafterweise abgerundet oder aus weichem Materialhergestellt. Das resorbierbare oder biologisch abbaubare Material ist zum Beispiel Nahtmaterial wie es zum Anlegen von chirurgischen Nähten verwendet wird und umfasst z.B. Kollagene, wie Catgut; Polyglykolsäure oder Polyglykonat. Ein derartiges Produkt ist beispielsweise Maxon (Polyglyconat) der Firma Davies + Geck, Gosport, Hampshire GB. Das Material soll einen solchen Vernetzungsgrad aufweisen, dass der Halte- und Positionierungsbalken genügend elastisch ist, damit ein problemloses Einführen möglich ist und er danach dennoch fest genug ist, damit er die vorübergehende Verschluss- und Haltefunktion ausüben kann. Weiter ist die Stärke und Struktur des resorbierbaren Nahtmaterials derart ausgewählt, dass der Abbau innerhalb von 6 Wochen stattfindet. Hierzu konnte zusätzlich eine zumindest partielle Umhüllung des Halte- und Positionierungsbalkens dienen. Das Material der Umhüllung könnten Produkte mit definierter biologischer Abbaubarkeit sein wie sie z.B. in Medikamenten mit verzögerter Wirkstoffabgabe zum Einsatz gelangen.

Die definitiven Abschlussorgane sollten in der Regel im wesentlichen eine abgerundete Form aufweisen, um die Mündungen der Eileiter in die Gebärmutter zu verschliessen. Es ist demzufolge vorteilhaft, wenn die definitiven Abschlussorgane, die nach der Rückbildung der Gebärmutter ihre Funktion übernehmen, diese Voraussetzungen erfüllen.

Da der Pessar zum Einsatz unmittelbar nach der Geburt bestimmt ist, muss das Röhrchen für seine Einführung nicht besonders dünn sein, wie dies bei normalen Pessaren der Fall ist, sondern es kann eine Dicke bis zu 3,6 cm aufweisen. Mit diesem Durchmesser kann das Röhrchen problemlos in den nach einer Geburt noch offenen Muttermund eingeführt werden. Dies hat den Vorteil, dass der Pessar zu seiner Einführung nicht oder nur wenig deformiert werden muss und trotzdem vor seiner Positionierung optimal geschützt ist.

Beim Einziehen des Pessars in das Röhrchen wurden bisher die beiden Äste, die vom Tragbalken weggespreizt sind, zusammengepresst und die etwa planen Flächen der beiden Abschlussorgane, die je am freien Ende der Äste befestigt sind, kamen aneinander oder übereinander zu liegen. Da aber am erfindungsgemässen Pessar der provisorische Halte- und Positionierungsbalken an den definitiven Abschlussorganen angebracht ist, ist eine Deformation etwas problematisch. Weil aber das Röhrchen zur Einführung bis zu 3,6 cm dick sein darf, kann der Pessar ohne Halte- und Positionierungsbalken praktisch unverformt in das Röhrchen eingeschoben werden. Nur noch die endständigen Teile des rechtwinklig zur Längsachse des Röhrchens angeordneten Halte- und Positionierungsbalkens ragt seitlich über dieses hinaus. Wegen seiner elastischen Deformierbarkeit können nun die beiden herausragenden Positionierungs-Enden des Halte- und Positionierungsbalkens während dem Einführungsvorgang derart abgebogen werden, dass sie aussen an das Röhrchen anliegen. Ist der Pessar im wesentlichen in seiner Position im Uterus, kann der Halte- und Positionierungsbalken wiederum seine ursprüngliche Form annehmen und seine Positionierungs-Enden können als provisorische Abschlussorgane für die Eileitermündungen fungieren. Der Halte- und Positionierungsbalken hält den Pessar während der Zurückbildung der Gebärmutter gleichzeitig in der korrekten Position. Nach ca. 6 Wochen hat die Gebärmutter wiederum ihre ursprüngliche Grösse und der Halte- und Positionierungsbalken ist abgebaut. Nun sind automatisch die definitiven Abschlussorgane an den Mündungen der Eileiter positioniert und der Pessar entspricht in Form und Funktion einem gewöhnlichen Okklusivpessar.

Es hat sich als vorteilhaft erwiesen, wenn das Röhrchen mit dem Pessar unmittelbar nach der Geburt in den geöffneten Muttermund eingeführt wird, was eine Verletzungsgefahr der Gebärmutter beim Einführen des Pessars vermeidet. Der abgerundete Halte- und Positionierungsbalken aus resorbierbarem elastischem Material hat dabei eine zusätzliche Schutzfunktion.

Die definitiven Abschlussorgane sind, wenn der Verschlussbalken abgebaut ist, an den Ästen derart angeordnet, dass diese auf der dem Tragbalken abgewandten Seite liegen. Dabei sind die Abschlussorgane so ausgebildet, dass deren im wesentlichen abgerundetes freies Ende schonend und weich im Bereich der Eileitermündungen gegen die Gebärmutterschleimhaut anstehen und damit diese Eileitermündungen dichtend abschliessen. Dank der Weichheit des Materials der Abschlussorgane ist eine Anpassung an die besonderen Gegebenheiten der Gebärmutter gegeben. Die beiden Abschlussorgane sind äusserst beweglich und weich und vorzugsweise mit einem Material auf Silikonbasis hergestellt. Der Tragbalken und die beiden Äste sind vorzugsweise einstückig hergestellt und bestehen aus einem beliebig steifen Material. Bewährt hat sich als geeigneter Kunststoff beispielsweise Polyethylen.

Die beiden Äste sind bei der Herstellung des Pessars so ausgeführt worden, dass sie sich bogenförmig vom Tragbalken wegerstrecken. Die Steifigkeit und Elastizität des gewählten Materiales bewirkt, dass sich die beiden Äste beim Einziehen des Pessars in das Röhrchen erforderlichenfalls zusammenfügen lassen und beim Ausstossen des Pessars aus dem Röhrchen, dank ihrer Elastizität wieder die bei der Herstellung vorgegebene Form einnehmen. Dadurch wird auch ein problemloses Entfernen des Pessars möglich.

In einer weiteren Ausführungsform des Pessars kann jeder Ast etwa mittig zwischen Tragbalken und Abschlussorgan einen Knick aufweisen.

Die genannten Äste mit den definitiven Abschlussorganen sind durch den Halte- und Positionierungsbalken verbunden. Gewöhnlich ist diese Verbindung spannungsfrei. In einer besonderen Ausführungsform der Erfindung kann der Halte- und Positionierungsbalken so befestigt sein, dass die beiden Äste gegenseitig in einer Spannung sind. Dadurch kann erreicht werden, dass erst nach dem vollständigen Abbau des Halte- und Positionierungsbalkens die Äste mit den definitiven Abschlussorganen die gewünschte endgültige Position mit einem grösseren oder kleineren gegenseitigen Abstand einnehmen können.

Anhand von Figuren sind bevorzugte Ausführungen der vorliegenden Erfindung beispielsweise näher beschrieben. Es zeigen
Fig. 1 den Erfindungsgegenstand mit Röhrchen mit auf den Ästen aufgesetztem Halte- und Positionierungsbalken in einer schaubildlichen Darstellung, und
Fig. 2 die Anordnung eines definitiven Abschlussorganes an einem freien Ende eines Astes.

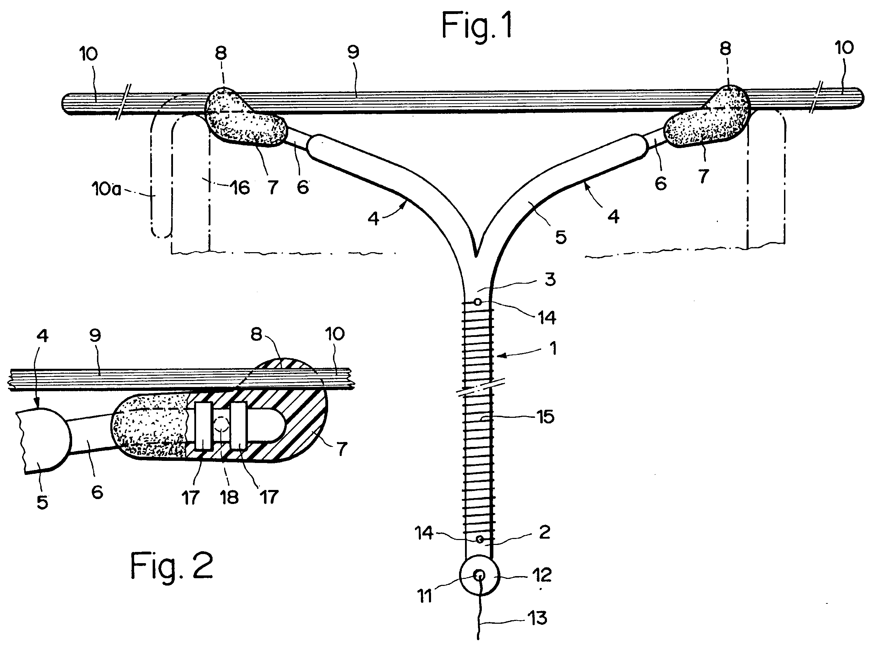

Beim dargestellten Ausführungsbeispiel gemäss der Fig. 1 weist der Pessar einen Tragbalken 1 auf, dessen eines in der Einführungsrichtung hinteres (distales) Ende 2 mit einer Öse 11 für das Durchschlaufen eines Ausziehfadens 13 versehen ist, während an seinem anderen, in der Einführrichtung vorderen (proximalen) Ende 3 zwei aus dem gleichen Material wie der Tragbalken bestehende Äste 4, die sich vom Tragbalken 1 bogenförmig wegerstrecken, angeordnet sind. Die Äste 4 sind federnd angeordnet, derart dass das Einführen und vor allem das Entfernen problemlos und ohne Verletzungsgefahr möglich ist. Das hintere Ende 12 ist kugel- oder tropfenförmig ausgebildet und kann somit nicht in die Schleimhaut der Gebärmutter eindringen. Der Tragbalken 1 ist vorzugsweise mit einem Kupferdraht 15 umwickelt, dessen empfängnisverhütende Wirkung ganz allgemein bekannt ist. Bei beiden Enden 2, 3 des Tragbalkens 1 sind Bohrungen 14 vorgesehen, in welchen die Drahtenden befestigt werden können.

Jeder Ast 4 umfasst einen mit 5 bezeichneten bogenförmig vom Tragbalken 1 sich wegerstreckenden Positionierungsteil, an dessen dem Tragbalken 1 angewandten Ende ein Fortsatz 6 anschliesst. Der Fortsatz 6 ist zum Anordnen eines definitiven Abschlussorganes 7 an seinem freien Ende bestimmt. Der Fortsatz 6 ist mit einem geringeren Durchmesser ausgeführt als der Positionierungsteil 5, wodurch erreicht wird, dass das definitve Abschlussorgan relativ zum Positionierungsteil 5 beweglich ist und sich nach dem Abbau des provisorischen Halte- und Positionierungsbalkens die günstigste Verschlussposition der Eileitermündungen aussuchen kann. Jedes Abschlussorgan 7, das im wesentlichen die Form eines kugeligen oder tropfenförmigen Körpers aufweist, besitzt ein integrales Haltemittel 8 für einen Halte- und Positionierungsbalken 9 aus resorbierbarem Nahtmaterial, wobei der Halte- und Positionierungsbalken bezüglich dem Tragbalken 1 rechtwinklig angeordnet ist; der Tragbalken 1 und der Halte- und Positionierungsbalken 9 bilden dabei die Form eines T. Weiter ist aus Fig. 1 die Position des Pessars in einem Röhrchen 16 ersichtlich, das als Hilfsmittel zum Einführen dient. Dabei ragen die Positionierungs-Enden 10 des Halte- und Positionierungsbalkens 9 über das Röhrchen 16 heraus. Beim Einführvorgang werden die Positionierungs-Enden 10 des flexiblen und elastischen Halte- und Positionierungsbalkens derart gekrümmt, dass sie eine Position 10a parallel zum Tragbalken 1 einnehmen. Das Rörchen 11 ist so ausgebildet, dass der Pessar zentral angeordet ist und leicht ausgestossen werden kann. Zur Vermeidung einer Verletzungsgefahr sind die Kanten des Röhrchens am proximalen Ende abgerundet.

Der Okklusivpessar wird ausgehend von einer Stellung wie angegegeben mittels dem Röhrchen 16 in die Gebärmutter eingeführt, wobei eine nicht dargestellte Stange dazu dient, den Pessar innerhalb des Röhrchens 16 vor bzw. aus dem Röhrchen hinauszuschieben. Wie bereits gesagt, schützt der aus dem Röhrchen vorstehende Teil des provisorischen Halte- und Positionierungsbalkens die Gebärmutter zusätzlich vor möglichen Verletzungen.

Sobald der Pessar innerhalb des Einführungsröhrchens 16 weit genug vorgeschoben ist, nimmt der Halte- und Positionierungsbalken dank der Elastizität seines Materials wiederum seine ursprüngliche gerade Form an, in welcher die Positionierungs-Enden 10, zu Beginne in einem Abstand von ca. 12 cm, schonend und weich im Bereich der Eileitermündungen gegen die Gebärmutterschleimhaut anstehen und damit diese Eileitermündungen schon unmittelbar nach einer Schwangerschaft dichtend abschliessen. In diesem Moment beginnt der biologische Abbau des resorbierbaren Materials des Halte- und Positionierungsbalkens im Milieu der Gebärmutter, wobei jedoch die Abschlussfunktion erhalten bleibt.

Dank der Weichheit des Materials der Abschlussorgane 7 sowie der Beweglichkeit der Fortsätze 6 wird nach dem Abbau des Halte- und Positionierungsbalkens 9 eine Anpassung der Abschlussorgane 7 an die besonderen Gegebenheiten der Gebärmutter ermöglicht.

Das Herausnehmen des Pessars erfolgt üblicherweise durch den durch die Öse 11 durchgeschlauften Faden 13, indem bei einem Zug auf diesen Faden der Tragbalken 1 und die durch elastische Deformation zusammenlegbaren Äste 4 des Pessars durch den Gebärmutterhals herausgezogen werden kann.

In der Fig. 2 ist gezeigt, wie das Abschlussorgan 7 am Fortsatz 6 befestigt ist. Der Fortsatz 6 ragt mit seinem, dem Positionierungsteil 5 des Astes 4 abgewandten freien Endes in das Abschlussorgan 7 hinein. In diesem Bereich sind am Fortsatz 6 Rippen 17 und/oder eine Bohrung 18 vorhanden, um das einwandfreie Halten des definitiven Abschlussorganes 7 zu gewährleisten. Dieses wird vorzugsweise an das genannte Ende des Fortsatzes 6 angespritzt. Anstelle der genannten Rippen könnten natürlich auch Nuten vorgesehen sein, die im gezeigten Ausführungsbeispiel nicht gezeichnet sind. Die definitiven Abschlussorgane weisen auf ihrer proximalen Seite im wesentlichen parallel zum Fortsatz 6 ein Durchgangsloch auf, welches dazu bestimmt ist, den provisorischen Halte- und Positionierungsbalken aufzunehmen, derart dass dieser zusammen mit dem Tragbalken 1 ein T bildet. Ist der provisorische Halte- und Positionierungsbalken durch biologischen Abbau entfernt übernimmt das definitive Abschlussorgan seine Funktion, wobei das Durchgangsloch in keiner Weise stört, da die abgerundeten Flächen auf der andern Seite des Abschlussorganes 7 die Schliessfunktion übernehmen.

Die beschriebenen Okklusivpessare gestatten, auf schonendste Weise unter Vermeidung jeder Reizung der Gebärmutterschleimhaut und unter Gewährung des freien Abflusses des Menstruationsblutes einen sicheren Abschluss der Eileitermündungen zu gewährleisten, wobei sie ebenso schmerzlos eingeführt wie herausgenommen werden können. Die Herstellung stellt keine weiteren Probleme dar, so dass die gezeigten Pessare einer weiten Verbreitung zuführbar sind.

## Patentansprüche

1. Okklusivpessar, der mittels eines Röhrchens (16) in die Gebärmutter eingeführt werden kann, enthaltend einen Tragbalken (1) und zwei an dessen in der Einführrichtung vorderem Ende (3) angeordnete Äste (4), die seitlich voneinander gespreizt sind, wobei die Äste (4) an ihren freien Enden je ein definitives Abschlussorgan (7) tragen, welche Abschlussorgane (7) dazu bestimmt und geeignet sind, die Mündungen der Eileiter in die Gebärmutter zu verschliessen, wobei die Äste (4) je einen vergleichsweise steifen Positionierungsteil (5) sowie ein am freien Ende des Positionierungsteiles (5) angeordneten dünnen, biegsamen und beweglichen Fortsatz (6) umfassen, an welchem Fortsatz (6) das definitive Abschlussorgan (7) angeordnet ist, wobei jedes Abschlussorgan (7) aus einem weichen Material gebildet ist, dadurch gekennzeichnet, dass an den beiden Abschlussorganen (7) ein provisorischer Halte- und Positionierungsbalken (9) mit Positionierungs-Enden (10) angeordnet ist, derart dass er zusammen mit dem Tragbalken (1) im wesentlichen die Form eines T bildet, wobei der Halte-und Positionierungsbalken aus resorbierbarem Material besteht.

2. Okklusivpessar nach Anspruch 1, dadurch gekennzeichnet, dass das resorbierbare Material resorbierbares Nahtmaterial aus Kollagen, Polyglykolsäure oder Polyglykonat ist.

3. Okklusivpessar nach Anspruch 1 oder 2, dadurch gekennzeichnet, dass die beiden definitiven Abschlussorgane (7) an ihrer proximalen Seite im wesentlich parallel zum Fortsatz (6) ausgerichtete Durchgangslöcher (8) aufweisen, die der Aufnahme des provisorischen Halte- und Positionierungsbalkens (9) dienen.

4. Okklusivpessar nach einem der Ansprüche 1 bis 3, dadurch gekennzeichnet, dass sich die beiden Äste (4) bogenförmig vom Tragbalken 1 wegerstrecken.

5. Okklusivpessar nach einem der Ansprüche 1 bis 3, dadurch gekennzeichnet, dass etwa mittig zwischen Tragbalken (1) und Abschlussorgan (7) jeder der Äste (4) eine abgeknickte Stelle aufweist.

6. Okklusivpessar nach einem der Ansprüche 1 bis 5 dadurch gekennzeichnet, dass der provisorische Halte- und Positionierungsbalken eine Länge von ca. 12 cm aufweist.

7. Okklusivpessar nach einem der Ansprüche 1 bis 6 dadurch gekennzeichnet, dass der Abstand der definitven Abschlussorgane 3 - 3,5 cm beträgt.

8. Okklusivpessar nach einem der Ansprüche 1 bis 7, dadurch gekennzeichnet, dass der Durchmesser des Röhrchens (16) ca 3,6 cm beträgt.

9. Okklusivpessar nach einem der Ansprüche 1 bis 8 dadurch gekennzeichnet, dass die Positionierungs-Enden 10 abgerundet sind und/oder aus weichem Material bestehen.

## Claims

1. Occlusive pessary, which can be inserted in the uterus by means of a small tube (16), containing a supporting stem (1) and, disposed at the front end (3) thereof in the direction of insertion, two branches (4), each bearing at their free ends a permanent closure member (7), which closure members (7) are intended and suitable for sealing the mouths of the Fallopian tubes into the uterus, each of the branches (4) comprising a comparatively stiff positioning portion (5) as well as a thin, flexible, and movable extension (6) disposed at the free end of the positioning portion (5), on which extension (6) the permanent closure member (7) is disposed, each closure member (7) being formed of a soft material, characterised in that a temporary holding and positioning rod (9) with positioning ends (10) is disposed on the two closure members (7) in such a way that together with the supporting stem (1) it substantially forms the shape of a T, the holding and positioning rod being made of material able to be resorbed.

2. Occlusive pessary according to claim 1, characterised in that the material able to be resorbed is resorbable suture material of collagen, polyglycolic acid, or polyglyconate.

3. Occlusive pessary according to claim 1 or 2, characterised in that the two permanent closure members (7) have on their proximal side, aligned substantially parallel to the extension (6), through bores (8) serving to receive the temporary holding and positioning rod (9).

4. Occlusive pessary according to one of the claims 1 to 3, characterised in that the two branches (4) extend arcuately away from the supporting stem (1).

5. Occlusive pessary according to one of the claims 1 to 3, characterised in that each of the branches (4) has a bent location about midway between the supporting stem (1) and closure member (7).

6. Occlusive pessary according to one of the claims 1 to 5, characterised in that the temporary holding and positioning rod has a length of about 12 cm.

7. Occlusive pessary according to one of the claims 1 to 6, characterised in that the spacing of the permanent closure members is from 3 to 3.5 cm.

8. Occlusive pessary according to one of the claims 1 to 7, characterised in that the diameter of the small tube (16) is about 3.6 cm.

9. Occlusive pessary according to one of the claims 1 to 8, characterised in that the positioning ends 10 are rounded and/or are made of sort material.

## Revendications

1. Pessaire occlusif pouvant être introduit dans l'utérus au moyen d'un petit tube (16) et comprenant une traverse porteuse (1) et deux branches (4) placées à son extrémité avant (3) vue dans le sens de l'introduction, ces branches (4) s'écartant latéralement l'une de l'autre et portant chacune, à leurs extrémités libres, un organe terminal (7) définitif, ces organes terminaux (7) étant destinés et aptes à obturer les orifices des trompes débouchant dans l'utérus, et ces branches (4) comprenant chacune une partie de positionnement (5) relativement rigide et un prolongement (6) mince, souple et mobile qui est placé à l'extrémité libre de la partie de positionnement (5), l'organe terminal définitif (7) étant placé sur ce prolongement (6) et chaque organe terminal (7) étant fait d'une matière molle, caractérisé en ce qu'une traverse provisoire de maintien et de positionnement (9) ayant des extrémités de positionnement (10) est située sur des deux organes terminaux (7), de telle façon qu'elle affecte sensiblement, avec la traverse porteuse (1), la forme d'un T, la traverse de maintien et de positionnement étant faite d'une matière qui se résorbe.

2. Pessaire occlusif selon la première revendication, caractérisé en ce que la matière pouvant être résorbée est une matière pour fils en collagène, en acide polyglycolique ou en polyglyconate.

3. Pessaire occlusif selon la revendication 1 ou 2, caractérisé en ce que les deux organes terminaux (7) définitifs présentent à leur extrémité proximale des trous traversants (8) sensiblement parallèles au prolongement (6) et servant à loger la traverse de maintien et de positionnement provisoire (9).

4. Pessaire occlusif selon l'une des revendications 1 à 3, caractérisé en ce que les deux branches (4) s'écartent de la traverse porteuse (1) en formant un arc.

5. Pessaire occlusif selon l'une des revendications 1 à 3, caractérisé en ce que chacune des branches (4) présente un coude à peu près à mi-chemin entre la traverse porteuse (1) et l'organe terminal (7).

6. Pessaire occlusif selon l'une des revendications 1 à 5, caractérisé en ce que la traverse provisoire de maintien et de positionnement présente une longueur d'environ 12 cm.

7. Pessaire occlusif selon l'une des revendications 1 à 6, caractérisé en ce que la distance entre les organes terminaux définitifs est de 3 à 3,5 cm.

8. Pessaire occlusif selon l'une des revendications 1 à 7, caractérisé en ce que le diamètre du petit tube (16) est d'environ 3,6 cm.

9. Pessaire occlusif selon l'une des revendications 1 à 8, caractérisé en ce que les extrémités de positionnement 10 sont arrondies ou sont faites d'une matière molle, ou les deux.
